(19) Europäisches Patentamt / European Patent Office / Office européen des brevets

(11) **EP 2 322 934 B1**

(12) **EUROPEAN PATENT SPECIFICATION**

(45) Date of publication and mention
of the grant of the patent:
**31.08.2016 Bulletin 2016/35**

(51) Int Cl.:
***G01N 33/68*** (2006.01)

(21) Application number: **10011289.5**

(22) Date of filing: **25.09.2006**

(54) **A Kit for diagnosing the Alzheimer's disease**

Kit zur Diagnose von Alzheimer

Kit pour le diagnostic de la maladie d'Alzheimer

(84) Designated Contracting States:
**AT BE BG CH CY CZ DE DK EE ES FI FR GB GR HU IE IS IT LI LT LU LV MC NL PL PT RO SE SI SK TR**

(30) Priority: **11.10.2005 US 246524**
**11.10.2005 PCT/US2005/036014**
**07.06.2006 PCT/US2006/022156**

(43) Date of publication of application:
**18.05.2011 Bulletin 2011/20**

(62) Document number(s) of the earlier application(s) in accordance with Art. 76 EPC:
**08020258.3 / 2 031 398**
**06825096.8 / 1 934 618**

(73) Proprietor: **Blanchette Rockefeller Neurosciences Institute**
**Morgantown, WV 26505-3409 (US)**

(72) Inventors:
• **Khan, Tapan Kumar**
**Morgantown, WV 26505 (US)**
• **Alkon, Daniel**
**Bethesda, MD 20817 (US)**

(74) Representative: **Kador & Partner**
**Corneliusstraße 15**
**80469 München (DE)**

(56) References cited:
**WO-A2-02/067764     WO-A2-03/102016**

• ZHAO W Q ET AL: "DYSFUNCTION OF MAP KINASE SIGNALING IN ALZHEIMER'S DISEASE", ABSTRACTS OF THE ANNUAL MEETING OF THE SOCIETY FOR NEUROSCIENCE, SOCIETY FOR NEUROSCIENCE, WASHINGTON, DC, US, vol. 27, no. 1, 10 November 2001 (2001-11-10), page 924, XP009027348, ISSN: 0190-5295
• ZHAO WEI-QIN ET AL: "MAP kinase signaling cascade dysfunction specific to Alzheimer's disease in fibroblasts", NEUROBIOLOGY OF DISEASE, BLACKWELL SCIENTIFIC PUBLICATIONS, OXFORD, GB, vol. 11, no. 1, 1 October 2002 (2002-10-01), pages 166-183, XP002525669, ISSN: 0969-9961, DOI: DOI:10.1006/NBDI.2002.0520
• KHAN TAPAN K ET AL: "An internally controlled peripheral biomarker for Alzheimer's disease: Erk1 and Erk2 responses to the inflammatory signal bradykinin", PROCEEDINGS OF THE NATIONAL ACADEMY OF SCIENCES OF THE UNITED STATES (PNAS), NATIONAL ACADEMY OF SCIENCE, US, vol. 103, no. 35, 1 August 2006 (2006-08-01), pages 13203-13207, XP002545475, ISSN: 0027-8424, DOI: DOI:10.1073/PNAS.0605411103

**Description**

**FIELD OF THE INVENTION**

The present invention relates to methods of diagnosing the presence or absence of Alzheimer's Disease in a subject by using a kit. The invention also relates to the use of said kit involving detecting alterations in the ratio of specific phosphorylated MAP kinase proteins in cells after stimulation with a protein kinase C activator. The Alzheimer's Disease-Specific Molecular Biomarkers (ADSMB) disclosed herein are useful for the diagnosis of Alzheimer's Disease.

**BACKGROUND OF THE INVENTION**

Perturbation of intracellular calcium homeostasis, increased levels of oxidative stress, and inflammatory mechanisms resulting in excitatory toxicity and neuronal death have been suggested to contribute to the pathogenesis of Alzheimer's Disease (AD) (Ito *et al.* 1994, Putney, 2000; Yoo *et al.,* 2000; Sheehan *et al.,* 1997; De Luigi *et al., 2001*; Anderson *et al.,* 2001; Remarque *et al.,* 2001). A number of AD-associated abnormalities in intracellular $Ca^{2+}$ levels and other cellular processes have derived from studies using bradykinin as a stimulus. As a potent inflammation mediator, bradykinin (BK) is produced by brain and peripheral cells under patho-physiological conditions such as trauma, stroke, _pain ischemia, and asthma (Regoli *et al.,* 1993; Bockmann & Paegelow, 2000; Ellis *et al.,* 1989; Kamiya *et al.,* 1993). By acting on the B2 bradykinin receptor (BK2bR), a G-protein-coupled receptor, BK triggers phosphatidylinositol (PI) turnover through activity of phospholipase C (PLC), which in turn produces inositol 1,4,5-trisphospate (IP3) that increases intracellular $Ca^{2+}$ release from the IP3-sensitive stores (Noda *et al.,* 1996; Venema *et al.,* 1998; Wassdal *et al.,* 1999; Cruzblanca *et al.,* 1998; Ricupero *et al.,* 1997; Pascale *et al.,* 1999). Through the same pathway, BK also triggers production of other proinflammatory cytokines through activation of mitogen-activated protein (MAP) kinases (Hayashi *et al.,* 2000; Schwaninger *et al.,* 1999; Phagoo *et al.,* 2001). Enhanced elevation of intracellular $Ca^{2+}$ levels has been found in AD brains as well as in AD peripheral cells in response to stimulation of bradykinin and inactivation of $K^+$ channels (Etcheberrigaray *et al.,* 1993, 1998; Hirashima *et al.,* 1996; Gibson *et al.,* 1996(a)).

Stimulation of PLC subsequent to BK2bR activation also leads to production of diacylglycerol which, along with increased intracellular $Ca^{2+}$, activates protein kinase C (PKC) isoforms. The BK-activated PLC/phospholipids-$Ca^{2+}$/PKC cascade interacts with the Ras/Raf signaling pathway, which activates extracellular signal-regulated kinases 1/2 (Erk 1 and Erk2, which are referred to together as "Erk1/2"), a subtype of the MAP kinase family (Berridge, 1984; BAssa *et al.,* 1999; Hayashi *et al.,* 2000; Blaukat *et al.,* 2000, Zhao et al. Neurobiology of Disease 11, 166-183, 2002). Erk1/2 receives signals from multiple signal transduction pathways and leads to cellular proliferation and differentiation by regulation of gene expression through a number of transcriptional factors, including AP-1, NF-κB, and cyclic AMP-responsive element binding protein (CREB). By acting as one of the major kinases, Erk2 phosphorylates tau at multiple serine/threonine sites including Ser-262 and Ser-356 (Reynolds *et al.,* 1993; Lu *et al.,* 1993). In addition, PKC-activated MAP kinase and BK receptor-associated pathways have been shown to regulate formation and secretion of the soluble form of amyloid precursor protein (sAPP) by different laboratories (Desdouits-Magnen *et al.,* 1998; Gasparini *et al.,* 2001; Nitsch *et al.,* 1994, 1995, 1996, 1998). These findings have suggested the possibility that BK-associated sAPP processing may be linked to the PKC-MAP kinase pathway. On the other hand, pathological conditions such as viral infections, increased oxidative stress, aberrant expression of APP, and exposure to APβ cause activation of MAP kinase (Rodems & Spector, 1998; McDonald *et al.,* 1998; Ekinci *et al.,* 1999; Grant *et al.,* 1999) and enhanced tau phosphorylation (Greenberg *et al.,* 1994; Ekinci & Shea, 1999; Knowles *et al.,* 1999). These effects implicate derangement of a MAP kinase signaling pathway(s) in the pathogenesis of AD.

Mitogen-activated protein kinases (such as Erk1 and Erk2) regulate phosphorylation of the microtubule associated protein tau and processing of the amyloid protein. both events critical to the pathophysiology of Azheimer's disease. Enhanced and prolonged Erk1/2 phosphorylation in response to bradykinin has been detected in fibroblasts of both familial and sporadic Alzheimer's Disease, but not age-matched controls (Zhao et al. Neurobiology of Disease 11, 166-183, 2002). Sustained Erk1/2 phosphorylation induced by bradykinin has previously been found in Alzheimer's Disease fibroblasts and is the subject of WO 02/067764.

WO 03/102016 A2 is directed to amyloid peptide inactivating enzymes to treat Alzheimer's Disease. Specifically, the document discloses the use of insulysin to hydrolyze alpha beta peptides which represents an alternative therapeutic approach to the treatment of Alzheimer's Disease. The use of certain agents, e.g. bradykinin peptides to increase insulysin enzyme activity and the use of a screening method to assess the effect of a compound on the activity of an amyloid peptide inactivating enzyme, is also described.

WO 02/067764 A2 discloses a method of diagnosing Alzheimer's disease in a patient comprises determining whether the phosphorylation level of an indicator protein in cells of the patient after stimulus with an activator compound is abnormally elevated as compared to a basal phosphorylation level, the indicator protein being e.g. Erk1/2 and the activator compound being e.g. bradykinin.

W.Q. Zhao et al. reports in Abstracts of the Annual Meeting of the Society for Neuroscience, Vol. 27, No.1, p. 924 on the dysfunction of MAP kinase signaling in Alzheimer's disease. Abnormally prolonged phosphorylation of Erk1/2 was detected in Alzheimer's disease (AD) fibroblasts in response to the stimulation by bradykinin, when compared to age-matched controls.

W.Q. Zhao et al. report in Neurobiology of Disease, Vol. 11, No. 1, p. 166-183 MAP kinase signaling cascade dysfunction specific to Alzheimer's disease in fibroblasts. The elevation of Erk ½ phosphorylation occurred immediately after bradykinin stimulation and required an IP-3-sensitive Ca2+ release as well as activation of protein kinace C and c-src as upstream events.

There exists a need for highly sensitive and highly specific tests to diagnose the presence or absence of Alzheimer's Disease. The present inventors have identified, for the first time, unique Alzheimer's Disease-specific molecular biomarkers useful for the diagnosis of the presence or absence of Alzheimer's Disease in a highly sensitive and highly specific manner compared to previously known diagnostic tests. Thus, the unique Alzheimer's Disease-specific molecular biomarkers disclosed herein serve as the basis for diagnostic methods having a high degree of sensitivity and specificity for the diagnosis of the presence or absence of Alzheimer's Disease.

## SUMMARY OF THE INVENTION

The present invention is directed to the use of a kit for diagnosing the presence or absence of Alzheimer's Disease in a subject, the kit comprising:

a) an agent that is a protein kinase C activator chosen from bradykinin; $\alpha$-APP modulator; bryostatin; bryologues; bombesin; cholecystokinin; thrombin; prostaglandin F2$\alpha$; vasopressin; 6-[N-decylamino]-4-hydroxymethylinole (DHI); 1,2-Dioctanoyl-sn-glycerol; farnesylthiotriazole (FTT); Gnidimacrin, *Stellera chamaejasme* L.; (-)-Indolactam V; Lipoxin A$_4$; Lyngbyatoxin A, *Micromonospora* sp.; Oleic acid; 1-Oleoyl-2-acetyl-sn-glycerol; 4$\alpha$-Phorbol-12,13-didecanoate; L-$\alpha$-Phosphatidylinositol-3,4-bisphosphate, Dipalmitoyl-, Pentaammonium Salt; L-$\alpha$-Phosphatidylinositol-4,5-bisphosphate, Dipalmitoyl-, Pentaammonium Salt; L-$\alpha$-Phosphatidylinositol-3,4,5-trisphosphate, Dipalmitoyl-, Heptaammonium Salt; 1-Stearoyl-2-arachidonoyl-sn-glycerol; Thymeleatoxin, *Thymelea hirsuta* L.; insulin; lysophosphatidylcholine; lipopolysaccharide; daunorubicin; and vanadyl sulfate;
b) an antibody specific for a phosphorylated first MAP kinase protein, wherein the first MAP kinase protein is Erk1;
c) an antibody specific for a phosphorylated second MAP kinase protein, wherein the second MAP kinase protein is Erk2;

wherein the use comprises:

i) contacting cells from said subject with said agent that is a protein kinase C activator;
ii) measuring the ratio of said phosphorylated first MAP kinase protein to said phosphorylated second MAP kinase protein, wherein said phosphorylated first and second MAP kinase proteins are obtained from said cells after said contacting step;
iii) measuring the ratio of said phosphorylated first MAP kinase protein to said phosphorylated second MAP kinase protein in cells from said subject that have not been contacted with the agent of step (i);
iv) subtracting the ratio obtained in step (iii) from the ratio obtained in step (ii); and
v) diagnosing the presence of Alzheimer's Disease in said subject based on the difference calculated in step (iv).

In certain embodiments of the invention, the cells that are used in the diagnostic assays are peripheral cells. In preferred embodiments, the cells may be skin cells, skin fibroblast cells, blood cells or buccal mucosa cells. In certain embodiments, the cells are not isolated from cerebral spinal fluid. In other preferred embodiments, the cells do not comprise cerebral spinal fluid. In still other preferred embodiments, the cells are not obtained by a spinal tap or lumbar puncture.

In certain embodiments of the diagnostic methods, a protein kinase C activator is contacted with cells in media comprising serum. In other preferred embodiments of the invention, a protein kinase C activator is contacted with said cells in serum-free media.

In certain embodiments of the diagnostic use of the invention, phosphorylated MAP kinase proteins are detected by immunoassay. In preferred embodiments of the invention, the immunoassay may be a radioimmunoassay, a Western blot assay, an immunofluoresence assay, an enzyme immunoassay, an immunoprecipitation assay, a chemiluminescence assay, an immunohistochemical assay, an immunoelectrophoresis assay, a dot blot assay, or a slot blot assay. In further preferred embodiments of the diagnostic use of the invention, protein arrays or peptide arrays or protein micro arrays may be employed in the diagnostic methods.

In preferred embodiments of the diagnostic use of the invention, the difference in the ratios calculated in step (iv) of claim 1 is diagnostic of Alzheimer's Disease in the subject if the difference is a positive value.

In other preferred embodiments of the diagnostic use of the invention, said difference is diagnostic of the absence of Alzheimer's Disease in the subject if the difference is a negative value or zero.

In certain embodiments of the invention, the kits for diagnosing Alzheimer's Disease in a subject may contain one or more protein kinase C activators selected from the group consisting of bradykinin, bryostatin, bombesin, cholecystokinin, thrombin, prostaglandin F2$\alpha$ and vasopressin. In preferred embodiments, the kits may contain any combination of the foregoing.

In the invention, the kits for diagnosing Alzheimer's Disease in a subject contain antibodies specific for phosphorylated Erk1 and phosphorylated Erk2. In preferred embodiments of the invention, the kit may contain anti-phospho-Erk1 antibody. In further preferred embodiments of the invention, the kit may contain an anti-phospho-Erk2 antibody. In preferred embodiments, the kits may contain any combination of the foregoing.

In certain embodiments of the invention, the kits for diagnosing Alzheimer's Disease may further comprise an amyloid beta peptide. In certain preferred embodiments, the amyloid beta peptide may be A$\beta$ (1-42).

## BRIEF DESCRIPTION OF THE FIGURES

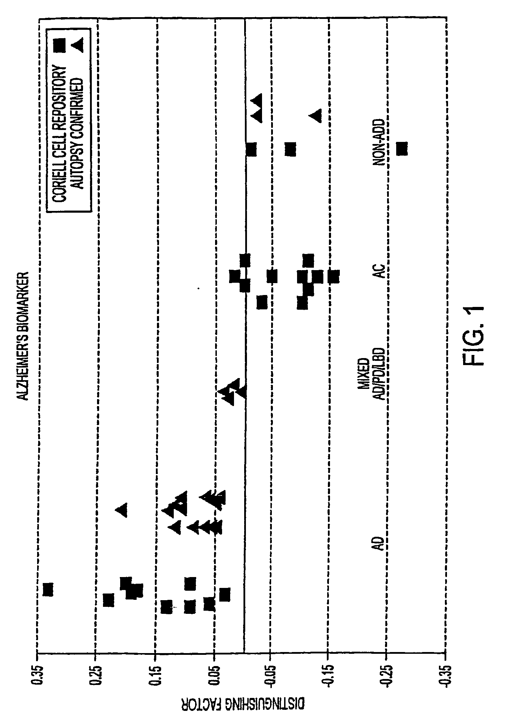

Figure 1 shows the results of determinations of Alzheimer's Disease-specific molecular biomarkers (ADSMB) in banked skin fibroblast cells obtained from the Coriell Cell Repository and in punch skin biopsy samples that were immediately placed in tissue culture and which were obtained from Autopsy confirmed subjects. AD refers to Alzheimer's Disease cells; Mixed AD/PD/LBD refers to cells taken from patients with mixed pathologies of Alzheimer's Disease, Parkinson's Disease and/or Loewi Body disease; AC refers to non-dementia age-matched control cells; Non-ADD refers to cells taken from subjects diagnosed with non-Alzheimer's Disease dementia (e.g. Huntington's disease or Parkinsons's disease or Clinical Schizophrenia). The Alzheimer's Disease-specific molecular biomarkers in AD cells tested was a positive value falling between greater than about 0.02 and less than about 0.4. The Alzheimer's Disease-specific molecular biomarker in Mixed AD/PD/LBD clustered together at very low positive values, i.e. less than about 0.02 or about 0.03. The Alzheimer's Disease-specific molecular biomarkers in non-dementia age-matched control cells were negative or very low positive values, i.e. less than about 0.01. The Alzheimer's Disease-specific molecular biomarkers in non-ADD cells were negative values. Alzheimer's Disease-specific molecular biomarkers were measured by determining the ratio of phosphorylated Erk1 to phosphorylated Erk2 in cells that had been stimulated with bradykinin minus the ratio of phosphorylated Erk1 to phosphorylated Erk2 in cells that were stimulated with media lacking bradykinin. This is expressed as the following: Alzheimer's Disease-specific molecular biomarker = $\{(pErk1/pErk2)_{bradykinin}\} - \{(pErk1/pErk2)_{vehicle}\}$. The ADSMB (noted as "Distinguishing Factor" (D.F.) in the figure) was plotted for four different categories of patients: Alzheimer's Disease (AD), mixed AD/PD/DLV (mixed diagnosis of Alzheimer's, Parkinson's and Lew body disease by autopsy confirmed) age matched control (AC) and non-AD dementia (Parkinson's disease and Huntington's disease) for Coriell cell repository and autopsy confirmed cell lines.

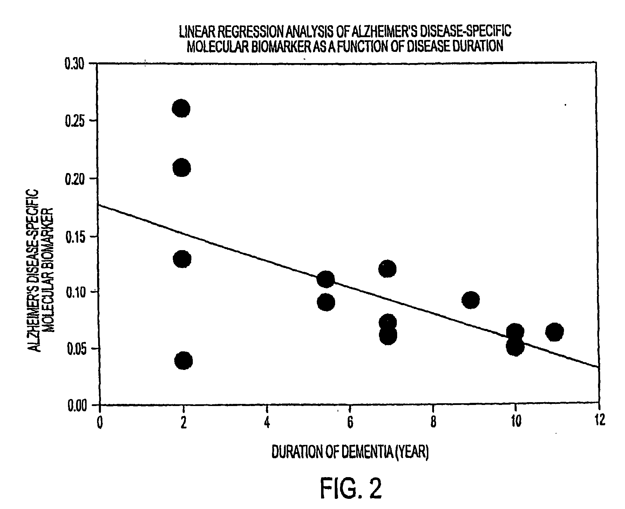

Figure 2 shows a linear regression analysis of Alzheimer's Disease-specific molecular biomarkers as a function of years of dementia. The linear regression shows a negative slope of approximately -0.01 indicating an inverse correlation between years of dementia and positive magnitude of the Alzheimer's Disease-specific molecular biomarker. As the years of dementia increases (i.e. as Alzheimer's Disease progresses) the Alzheimer's Disease-specific molecular biomarker becomes a less positive numerical value. Measurement of the Alzheimer's Disease-specific molecular biomarker allows for early diagnosis of Alzheimer's Disease because a more highly positive value is indicative of early stages of the disease. Alzheimer's Disease-specific molecular biomarkers were measured by determining the ratio of phosphorylated Erk1 to phosphorylated Erk2 in cells that had been stimulated with bradykinin minus the ratio of phosphorylated Erk1 to phosphorylated Erk2 in cells that were stimulated with media lacking bradykinin. This is expressed as the following: Alzheimer's Disease-specific molecular biomarker = $\{(pErk1/pErk2)_{bradykinin}\} - \{(pErk1/pErk2)_{vehicle}\}$. Linear regression analysis of Alzheimer's Disease-specific molecular biomarker (ADSMB) as a function of disease duration of autopsy confirmed cases. Alzheimer's Disease-specific molecular biomarker is more effective in early years of the disease. This shows that the present method is more effective in early diagnosis of Alzheimer's Disease.

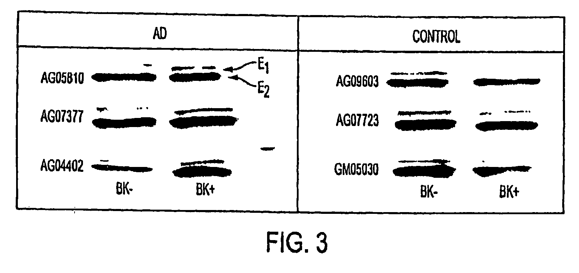

Figure 3 shows western blot data of p-Erk1/2 (phosphorylated Erk1 and Erk2) after bradykinin (BK+) treatment and vehicle (DMSO, without bradykinin, BK-) for AD and control cell lines. For bradykinin treatment (BK+), serum free (24 hrs) cells were treated with 10 nM bradykinin for 10 min at 37°C. The corresponding vehicle treatment (BK-), serum free (24 hrs) cells were treated with the same amount of DMSO (without BK) for 10 min at 37°C. After 10 min P-Erk1/2 bands were darker for BK+ than that of BK- treatment for AD cell line, but it is reverse for control cell lines. This shows that BK induced activation of Erk was higher for AD cell lines.

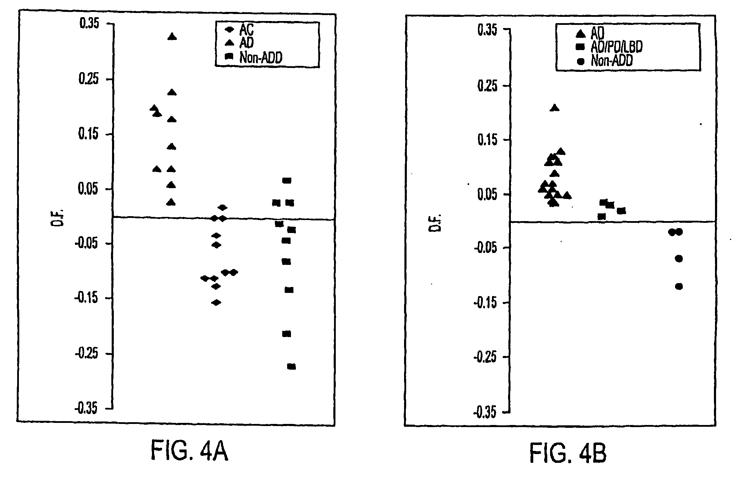

Figures 4A and 4B show Alzheimer's Disease-Specific Molecular Biomarker (ADSMB) (noted as the "Distinguishing Factor" (D.F.) in the figure) calculated as discussed herein. ADSMB was plotted for AD (Alzheimer's Disease), AC (age matched control) and non-ADD (non AD dementia, e.g. Parkinson's disease Lewy body disease) cell lines from Coriell repository (A) (Coriell Institute of Medical Research, Camden, New Jersey) and cell lines provided by Neurologic Inc. (autopsy confirmed) (B). The results show that ADSMB for AD cases was consistently higher than for AC and non-ADD cases.

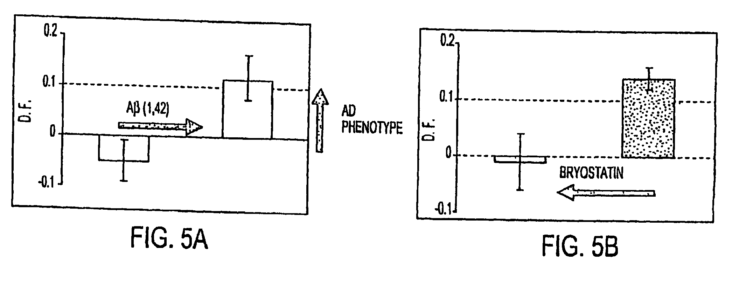

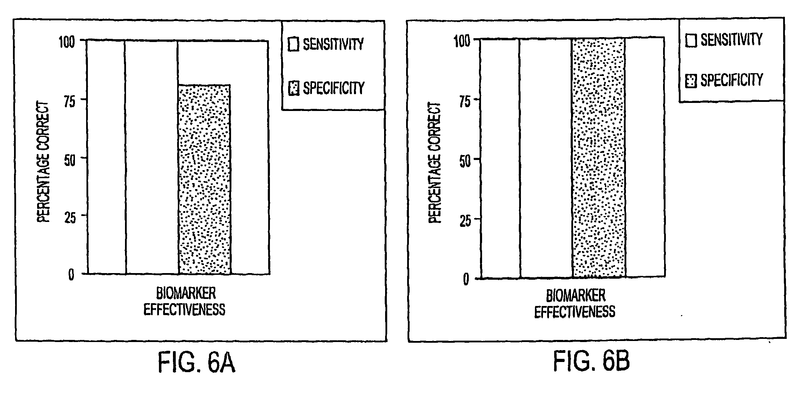

Figures 5A and 5B show soluble Aβ induces and bryostatin treatment reverses Alzheimer's phenotype of human fibroblast. (A) Alzheimer's Disease-Specific Molecular Biomarker (noted as "Distinguishing Factor" (D.F.) in the figure) was measured for control (non-AD) cell lines (AG07723, AG11363, AG09977, AG09555 and AG09878) as described herein and found small and negative. After 1.0 μM Aβ-42 treatment (ADSMB) was measured again as described and found higher and positive. This shows that the bradykinin induced, activated Erk1/ Erk2 ratio becomes higher after 1.0 μM Aβ(1-42) treatment. AC cell lines behave like AD phenotype after Aβ(1-42) treatment. (B) ADSMB ("Distinguishing Factor" (D.F.)) was measured after 1.0 μM A (1-42) treatment for 24 hrs for AC cell lines. The ADSMB values were higher and positive as found earlier. The same cell lines were treated first with 1.0 μM Aβ(1-42) for 24 hrs and followed by 0.1 nM bryostatin treatment for 20 min. The ADSMB (D.F.) values were again measured and found small and negative. This shows that soluble Aβ-induced changes can be reversed by bryostatin therapy. Figures 6A and 6B shows a decision matrix analysis of the ADSMB. Sensitivity and specificity of the biomarker are plotted to show the effectiveness to detect the disease for Coriell cell repository (A) and autopsy confirmed (B) cells.

## DETAILED DESCRIPTION

The present invention relates, in certain aspects, to the use of a kit capable of diagnosing the presence or absence of Alzheimer's Disease in human cells taken from subjects that have been identified for testing and diagnosis. The diagnosis is based upon the discovery of unique Alzheimer's Disease-specific molecular biomarkers. In certain aspects, the invention is directed to methods for determining or confirming the presence or absence of Alzheimer's Disease in a subject or in samples taken from a subject.

The inventors have found a unique molecular biomarker for Alzheimer's Disease using peripheral (non-CNS) tissue that is useful in diagnostic assays that are highly sensitive and highly specific for the diagnosis of Alzheimer's Disease. A great advantage of the instant invention is that the tissue used in the assays and methods disclosed herein may be obtained from subjects using minimally invasive procedures, i.e., without the use of a spinal tap. Thus, one aspect of the invention is directed to an assay or test for the early detection of Alzheimer's Disease in a subject in which an internally controlled ratio of Erk1 phosphorylation to Erk2 phosphorylation, which is induced by a protein kinase C activator (such as bradykinin), is measured with specific antibodies using a baseline normalization response to growth media in human cells, such as skin fibroblasts, or other peripheral cells such as blood cells.

In the use of the invention, the cells that are taken from the individual or patient can be any viable cells. Preferably, they are skin fibroblasts, but any other peripheral tissue cell (i.e. tissue cells outside of the central nervous system) may be used in the tests of this invention if such cells are more convenient to obtain or process. Other suitable cells include, but are not limited to, blood cells such as erythrocytes and lymphocytes, buccal mucosal cells, nerve cells such as olfactory neurons, cerebrospinal fluid, urine and any other peripheral cell type. In addition, the cells used for purposes of comparison do not necessarily have to be from healthy donors.

The cells may be fresh or may be cultured (see, U.S. Patent No. 6,107,050,). In a specific embodiment, a punch skin biopsy can be used to obtain skin fibroblasts from a subject. These fibroblasts are analyzed directly using the techniques described herein or introduced into cell culture conditions. The resulting cultured fibroblasts are then analyzed as described in the examples and throughout the specification. Other steps may be required to prepare other types of cells which might be used for analysis such as buccal mucosal cells, nerve cells such as olfactory cells, blood cells such as erythrocytes and lymphocytes, etc. For example, blood cells can be easily obtained by drawing blood from peripheral veins. Cells can then be separated by standard procedures (e.g. using a cell sorter, centrifugation, etc.) and later analyzed.

Thus, the present invention relates, in certain aspects, to methods for the diagnosis and treatment of Alzheimer's Disease in a subject. In certain embodiments, the diagnostic methods of the invention are based on measuring the ratio of two specific and distinct phosphorylated MAP kinase proteins in cells taken from a subject which have been stimulated with an agent that is a protein kinase C activator. The invention is also directed, in certain embodiments, to the use of kits containing reagents useful for the detection or diagnosis of Alzheimer's Disease.

I. Definitions

As used herein, the term "sensitivity" in the context of medical screening and diagnosis, means the proportion of affected individuals who give a positive test result for the disease that the test is intended to reveal, i.e., true positive results divided by total true positive and false negative results, usually expressed as a percentage.

As used herein, the term "specificity" in the context of medical screening and diagnosis, means the proportion of individuals with negative test results for the disease that the test is intended to reveal, i.e., true negative results as a proportion of the total of true negative and false-positive results, usually expressed as a percentage.

As used herein, the term " highly sensitive" means a diagnostic method that is greater than or equal to about 50% sensitive, or about 55% sensitive, or about 60% sensitive, or about 65% sensitive, or about 70% sensitive, or about 75% sensitive, or about 80% sensitive, or about 85% sensitive, or about 90% sensitive, or about 95% sensitive, or about 96% sensitive, or about 97% sensitive, or about 98% sensitive, or about 99% sensitive or about 100% sensitive.

As used herein, the term "highly specific" means a diagnostic method that is greater than or equal to about 50% specific, or about 55% specific, or about 60% specific, or about 65% specific, or about 70% specific, or about 75% specific, or about 80% specific, or about 85% specific, or about 90% specific, or about 95% specific, or about 96% specific, or about 97% specific, or about 98% specific, or about 99% specific or about 100% specific.

As used herein, "sequence variants" are proteins that are related to each other both structurally and functionally. In certain embodiments, sequence variants are proteins that share structural similarity at the level of amino acid sequence and share functional attributes at the level of enzymatic activity. In certain embodiments, sequence variants are MAP kinase proteins that catalyze the phosphorylation of other proteins.

As used herein, the "absence of Alzheimer's Disease" means that a subject or cells taken from a subject do not exhibit a measurable or detectable Alzheimer's Disease phenotype.

As used herein, an "Alzheimer's Disease phenotype" in a subject or a cell sample includes but is not limited to an Alzheimer's Disease-specific molecular biomarker having a positive value greater than zero.

As used herein, an "Amyloid Beta Peptide" is any fragment of the Amyloid Beta Peptide or a full-length Amyloid Beta Peptide.

II. Methods of Diagnosing Alzheimer's Disease

The present invention is directed, in certain embodiments, to methods of diagnosing the presence or absence of Alzheimer's Disease. In certain specific embodiments, the diagnostic assays disclosed herein may be carried out *in vitro* or *in vivo*. In a specific embodiment, the protein kinase C activator is bradykinin. The ratio of specific phosphorylated MAP kinase proteins is the ratio of phosphorylated Erk1 to phosphorylated Erk2, which is calculated by dividing the relative or normalized amount of phosphorylated Erk1 by the relative or normalized amount of phosphorylated Erk2.

Alzheimer's Disease-Specific Molecular Biomarkers

The diagnostic methods which are disclosed herein are based upon the discovery by the inventors of a unique molecular biomarker for Alzheimer's Disease. The numerical value of the Alzheimer's Disease-specific molecular biomarker will depend on certain variables, such as, for example, the cells used in the assay, the protein kinase C activator used in the assay and the specific MAP kinase proteins that are targeted for measurement of phosphorylation ratios.

In a specific embodiment, the Alzheimer's Disease-specific molecular biomarker may be measured by determining the ratio of phosphorylated Erk1 to phosphorylated Erk2 in cells that have been stimulated by a protein kinase C activator and subtracting from this the ratio of phosphorylated Erk1 to phosphorylated Erk2 in cells that have been stimulated with a control solution (vehicle) that lacks the protein kinase C activator. In certain embodiments, if the difference is greater than zero, i.e. a positive value, this is diagnostic of Alzheimer's Disease. In further preferred embodiments, if the difference is less than or equal to zero, this is indicative of the absence of Alzheimer's Disease.

In other embodiments, the Alzheimer's Disease-specific molecular biomarkers of the present invention are measured by determining the ratio of two phosphorylated MAP kinase proteins after stimulation of cells with a protein kinase C activator. The molecular biomarker may be measured by determining the ratio of a first phosphorylated MAP kinase protein to a phosphorylated second MAP kinase protein in cells that have been stimulated by a protein kinase C activator and subtracting from this the ratio of phosphorylated first MAP kinase protein to phosphorylated second MAP kinase protein in cells that have been stimulated with a control solution (vehicle) that lacks the protein kinase C activator. In certain preferred embodiments, if the difference is greater than zero, i.e. a positive value, this is diagnostic of Alzheimer's Disease. In further preferred embodiments, if the difference is less than or equal to zero, this is indicative of the absence of Alzheimer's Disease.

In certain embodiments, the Alzheimer's Disease-specific molecular biomarker is a positive numerical value in cell samples taken from patients diagnosed with Alzheimer's Disease (AD cells). In certain preferred embodiments, when the Alzheimer's Disease-specific molecular biomarker is measured by determining ratios of phosphorylated Erk1 to phosphorylated Erk2 in AD cells that have been stimulated with bradykinin, the positive numerical values for the Alzheimer's Disease-specific molecular biomarker in AD cells may range from about zero to about 0.5.

In certain embodiments, the Alzheimer's Disease-specific molecular biomarker is a negative numerical value when measured in cells taken from subjects diagnosed with non-Alzheimer's Disease dementia (non-ADD cells), such as, for example, Parkinson's disease or Huntington's disease or Clinical Schizophrenia. In certain preferred embodiments, when the Alzheimer's Disease-specific molecular biomarker is measured by determining ratios of phosphorylated Erk1 to phosphorylated Erk2 in non ADD cells that have been stimulated with bradykinin, the negative numerical values may range from about zero to about -0.2 or about -0.3.

In certain embodiments, the Alzheimer's Disease-specific molecular biomarker may be a negative numerical value, zero or very low positive numerical value in cell samples from age-matched control cells (AC cells) taken from patients who do not have Alzheimer's Disease. When the Alzheimer's Disease-specific molecular biomarker is measured by determining ratios of phosphorylated Erk1 to phosphorylated Erk2 in AC cells that have been stimulated with bradykinin, the Alzheimer's Disease-specific molecular biomarker in AC cells may range from less than about 0.05 to about -0.2.

In certain embodiments of the invention, the Alzheimer's Disease-specific molecular biomarkers may be measured by calculating the ratio of phosphorylated Erk1 to phosphorylated Erk2 in cells that have been stimulated with bradykinin minus the ratio of phosphorylated Erk1 to phosphorylated Erk2 in cells that have stimulated with a solution lacking bradykinin. This is expressed as the following: Alzheimer's Disease-specific molecular biomarker = $\{(\text{pErk1/pErk2})_{\text{bradykinin}}\} - \{(\text{pErk1/pErk2})_{\text{vehicle}}\}$.

Protein Kinase C Activators

Protein kinase C activators that are specifically contemplated for use in the diagnostic methods, and the use of kits of the instant invention include: Bradykinin; $\alpha$-APP modulator; Bryostatin 1; Bryostatin 2; DHI; 1,2-Dioctanoyl-sn-glycerol; FTT; Gnidimacrin, *Stellera chamaejasme* L.; (-)-Indolactam V; Lipoxin A$_4$; Lyngbyatoxin A, *Micromonospora* sp.; Oleic acid; 1-Oleoyl-2-acetyl-sn-glycerol; 4 $\alpha$-Phorbol; Phorbol-12,13-dibutyrate; Phorbol-12,13-didecanoate; 4$\alpha$-Phorbol-12,13- didecanoate; Phorbol-12-myristate-13-acetate; L-$\alpha$-Phosphatidylinositol-3,4-bisphosphate, Dipalmitoyl-, Pentaammonium Salt; L-$\alpha$-Phosphatidylinositol-4,5-bisphosphate, Dipalmitoyl-, Pentaammonium Salt; L-$\alpha$-Phosphatidylinositol-3,4,5-trisphosphate, Dipalmitoyl-, Heptaammonium Salt; 1-Stearoyl-2-arachidonoyl-sn-glycerol; Thymeleatoxin, *Thymelea hirsute L.*; insulin, phorbol esters, lysophosphatidylcholine, lipopolysaccharide, anthracycline dannorubicin and vanadyl sulfate. Also included are compounds known as "bryologues." Bryologues are described, for example, in Wender et al. Organic letters (United States) May 12, 2005 ,7 (10) p1995-8; Wender et al. Organic letters (United States) Mar 17 2005, 7 (6) p1177-80; Wender et al. Journal of Medicinal Chemistry (United States) Dec 16 2004, 47 (26) p6638-44. A protein kinase C activator may be used alone or in combination with any other protein kinase C activator in the diagnostic methods, and use of kits disclosed herein.

Bradykinin is a potent vasoactive nonapeptide that is generated in the course of various inflammatory conditions. Bradykinin binds to and activates specific cell membrane bradykinin receptor(s), thereby triggering a cascade of intracellular events leading to the phosphorylation of proteins known as "mitogen activated protein kinase" (MAPK). Phosphorylation of protein, the addition of a phosphate group to a Ser, Thr, or Tyr residue, is mediated by a large number of enzymes known collectively as protein kinases. Phosphorylation normally modifies the function of, and usually activates, a protein. Homeostasis requires that phosphorylation be a transient process, which is reversed by phosphatase enzymes that dephosphorylate the substrate. Any aberration in phosphorylation or dephosphorylation may disrupt biochemical pathways and cellular functions. Such disruptions may be the basis for certain brain diseases.

Measuring or Detecting Levels of Phosphorylated Proteins

The methods of diagnosing Alzheimer's Disease herein disclosed depend on measuring the Alzheimer's Disease-specific molecular biomarkers as used in the present invention.

In a preferred embodiment, the level of phosphorylated protein present in cells is detected by Western blotting. Protein levels of phosphorylated Erk1 or phosphorylated Erk2 can be measured in fibroblasts using anti-Erk1, anti-Erk2, anti-phospho-Erk1 and anti-phospho-Erk2 antibodies (Cell Signaling Technology). Levels of a different protein may also be measured in the same sample as a reference protein for normalization. Examples of possible reference proteins include, but are not limited to, annexin-II or actin.

In one embodiment, ELISA is performed according to the following procedures: 1) Add fibroblast cell lysates after treatment in duplicates or triplicates to a 96-well microplate that is previously coated with an anti-Erk antibody. 2) Incubate samples in microplate wells at room temperature for about 2 hours. 3) Aspirate samples and wash wells with a phosphate buffered saline (PBS)-based washing buffer. 4) Add working dilution of an anti-phospho-Erk1/2, or an anti-regular Erk1/2 antibody to each well, and incubate at room temperature for about 1 hour. 5) Aspirate and wash well with washing buffer. 6) Add a working dilution of a secondary antibody conjugated with horseradish peroxidase (HRP) to each well and incubate well at room temperature for about 30 min. 7) Aspirate and wash well with washing buffer. 8) Add stabilized Chromogen such as diaminobenzidine (DAB) and incubate at room temperature for about 30 min. 9) Add stop solution and measure the absorbance at 450 nm. Phosphorylation of Erk1/2 is assessed after normalization: NR $= A_P/A_R$. Where NR = the normalized ratio; $A_P$ is absorbance values for phospho-Erk1/2; and $A_R$ is absorbance for the total (regular) Erk1/2.

In a preferred embodiment, phosphorylation of Erk1/2 is assayed on Western blots using an anti-phospho-Erk1/2 antibody. Levels of the immunoreactive signals for phosphorylated Erk1/2 are quantified via densitometric scan. The mean density of the phospho-Erk1/2 signals are normalized with the mean density of total Erk1/2 signals that are detected from the same cell lysate samples with an anti-regular Erk1/2 antibody on a separate Western blot. The formula for normalization is: $NR = D_P/D_R$. Where NR (normalized ratio) represents Erk1/2 phosphorylation extent; $D_P$ is the mean density for phospho-Erk1/2, and $D_R$ is the mean density for the total amount of Erk1/2 detected on a Western blot from the same sample.

Immunoassays used in the present invention for the detection of proteins may be immunofluorescent assays, radioimmunoassays, Western blot assays, enzyme immunoassay, immuno-precipitation, chemiluminescent assay, immunohistochemical assay, dot or slot blot assay. (In "Principles and Practice of Immunoassay" (1991) Christopher P. Price and David J. Neoman (eds), Stockton Press, New York, New York, Ausubel et al. (eds) (1987) in "Current Protocols in Molecular Biology" John Wiley and Sons, New York, New York). Detection may be by colorimetric or radioactive methods or any other conventional methods known to those having skill in the art. Standard techniques known in the art for ELISA are described in Methods in Immunodiagnosis, 2nd Edition, Rose and Bigazzi, eds., John Wiley and Sons, New York 1980 and Campbell et al., Methods of Immunology, W.A. Benjamin, Inc., 1964. Such assays may be direct, indirect, competitive, or noncompetitive immunoassays as described in the art (In "Principles and Practice of Immunoassay" (1991) Christopher P. Price and David J. Neoman (eds), Stockton Pres, NY, NY; Oellirich, M. 1984. J. Clin. Chem. Clin. Biochem. 22: 895-904 Ausubel, et al. (eds) 1987 in Current Protocols in Molecular Biology, John Wiley and Sons, New York, New York.

Cell Types, Protein Isolation and Antibodies

As stated previously, the cells taken from the patient being diagnosed may be any cell. Examples of cells that may be used include, but are not limited to, skin cells, skin fibroblasts, buccal mucosal cells, blood cells, such as erythrocytes, lymphocytes and lymphoblastoid cells, and nerve cells and any other cell expressing the Erk1/2 protein. Necropsy samples and pathology samples may also be used. Tissues comprising these cells may also be used, including brain tissue or brain cells. The cells may be fresh, cultured or frozen. Protein samples isolated from the cells or tissues may be used immediately in the diagnostic assay or use or frozen for later use. In a preferred embodiment fibroblast cells are used. Fibroblast cells may be obtained by a skin punch biopsy.

Proteins may be isolated from the cells by conventional methods known to one of skill in the art. In a preferred method, cells isolated from a patient are washed and pelleted in phosphate buffered saline (PBS). Pellets are then washed with "homogenization buffer" comprising 50 nM NaF, 1mM EDTA, 1 mM EGTA, 20 µg/ml leupeptin, 50 µg/ml pepstatin, 10 mM TRIS-HCl, pH = 7.4, and pelleted by centrifugation. The supernatant is discarded, and "homogenization buffer" is added to the pellet followed by sonication of the pellet. The protein extract may be used fresh or stored at -80°C for later analysis.

In the use of the invention, the antibodies used in the disclosed immunoassays may be monoclonal or polyclonal in origin. The phosphorylated and non-phosphorylated Erk1/2 protein or portions thereof used to generate the antibodies may be from natural or recombinant sources or generated by chemical synthesis. Natural Erk1/2 proteins can be isolated from biological samples by conventional methods. Examples of biological samples that may be used to isolate the Erk1/2 protein include, but are not limited to, skin cells, such as, fibroblasts, fibroblast cell lines, such as Alzheimer's Disease fibroblast cell lines and control fibroblast cell lines which are commercially available through Coriell Cell Repositories, (Camden, N.J.) and listed in the National Institute of Aging 1991 Catalog of Cell Lines, National Institute of General Medical Sciences 1992/1993 Catalog of Cell Lines [(NIH Publication 92-2011 (1992)].

III. Kits for the Diagnosis of Alzheimer's Disease

This invention relates to the use of kits which may be utilized in performing any of the diagnostic tests described above. The kits comprise antibodies which recognize regular Erk1/2 (unphosphorylated Erk1 or unphosphorylated Erk2) or phosphorylated Erk1/2 (phosphorylated Erk1 or phosphorylated Erk2). The kits also contain any one or more of the protein kinase C activators disclosed herein (such as, for example, bradykinin or bryostatin). Antibodies may be polyclonal or monoclonal. The kits may contain instruments, buffers and storage containers necessary to perform one or more biopsies, such as punch skin biopsies. The kits may also contain instructions relating to the determination of the ratios used to identify the Alzheimer's Disease-specific molecular biomarkers used in the instant invention as well as the use of the antibodies or other constituents in the diagnostic tests. The instructions may also describe the procedures for performing a biopsy, such as a punch skin biopsy. The kits may also contain other reagents for carrying out the diagnostic tests such as antibodies for the detection of reference proteins used for normalization. Examples of antibodies that recognize possible reference proteins include, but are not limited to, antibodies that recognize annexin-II or actin. The kits may also include buffers, secondary antibodies, control cells.

Alzheimer's Disease-specific molecular biomarkers, in certain embodiments, are measured by determining the ratio of phosphorylated Erk1 to phosphorylated Erk2 in cells that have been stimulated with bradykinin minus the ratio of phosphorylated Erk1 to phosphorylated Erk2 in cells that have been stimulated with media lacking bradykinin. This is expressed as the following: Alzheimer's Disease-specific molecular biomarker = $\{(pErk1/pErk2)_{bradykinin}\}$ - $\{(pErk1/pErk2)_{vehicle}\}$.

VI. Amyloid Beta Peptide

The terms "amyloid beta peptide", "beta amyloid protein", "beta amyloid peptide", "beta amyloid", "A. beta" and "A. beta peptide" are used interchangeably herein. In some forms, an amyloid beta peptide (e.g., A. beta 39, A. beta 40, A. beta 41, A. beta 42 and A. beta 43) is an about 4-kDa internal fragment of 39-43 amino acids of the larger transmembrane glycoprotein termed Amyloid Precursor Protein (APP). Multiple isoforms of APP exist, for example $APP^{695}$, $APP^{751}$, and $APP^{770}$. Examples of specific isotypes of APP which are currently known to exist in humans are the 695 amino acid polypeptide described by Kang et. al. (1987) Nature 325:733-736 which is designated as the "normal" APP; the 751 amino acid polypeptide described by Ponte et al. (1988) Nature 331:525-527 (1988) and Tanzi et al. (1988) Nature 331:528-530; and the 770-amino acid polypeptide described by Kitaguchi et. al. (1988) Nature 331:530-532. As a result of proteolytic processing of APP by different secretase enzymes *in vivo* or *in situ*, A. beta is found in both a "short form", 40 amino acids in length, and a " long form" , ranging from 42-43 amino acids in length. Part of the hydrophobic domain of APP is found at the carboxy end of A. beta, and may account for the ability of A. beta to aggregate, particularly in the case of the long form. A. beta. peptide can be found in, or purified from, the body fluids of humans and other mammals, e.g. cerebrospinal fluid, including both normal individuals and individuals suffering from amyloidogenic disorders.

The terms "amyloid beta peptide", "beta amyloid protein", "beta amyloid peptide", "beta "amyloid", "A. beta" and "A. beta peptide" include peptides resulting from secretase cleavage of APP and synthetic peptides having the same or essentially the same sequence as the cleavage products. A. beta. peptides of the invention can be derived from a variety of sources, for example, tissues, cell lines, or body fluids (*e.g.* sera or cerebrospinal fluid). For example, an A. beta can be derived from APP-expressing cells such as Chinese hamster ovary (CHO) cells as described, for example, in Walsh et al., (2002), Nature, 416, pp 535-539. An A. beta. preparation can be derived from tissue sources using methods previously described (*see, e.g.,* Johnson-Wood et al., (1997), Proc. Natl. Acad. Sci. USA 94:1550). Alternatively, A. beta. peptides can be synthesized using methods which are well known to those in the art. See, for example, Fields et al., Synthetic Peptides: A User's Guide, ed. Grant, W.H. Freeman & Co., New York, N.Y., 1992, p 77). Hence, peptides can be synthesized using the automated Merrifield techniques of solid phase synthesis with the a-amino group protected by either t-Boc or F-moc chemistry using side chain protected amino acids on, for example, an Applied Biosystems Peptide Synthesizer Model 430A or 431. Longer peptide antigens can be synthesized using well known recombinant DNA techniques. For example, a polynucleotide encoding the peptide or fusion peptide can be synthesized or molecularly cloned and inserted in a suitable expression vector for the transfection and heterologous expression by a suitable host cell. A. beta. peptide also refers to related A. beta sequences that results from mutations in the A. beta. region of the normal gene.

The A. beta - induced abnormality of the Erk 1/2 index (Alzheimer's Disease-specific molecular biomarker) may be used as a confirmatory test for either the presence or absence of Alzheimer's disease. Namely, a negative Amyloid Beta-Index response indicates the presence of disease, while a positive response indicates the absence of disease. That is, if an Alzheimer's Disease phenotype is induced in cells upon incubation or contact with an Amyloid Beta Peptide, this is indicative of the absence of the disease in the test cells or subject being tested. In contrast, if no or little change in an Alzheimer's Disease-specific molecular biomarker is induced in cells upon incubation or contact with an Amyloid Beta Peptide, this is indicative of the presence of Alzheimer's Disease in the test cells or subject being tested.

While amyloid beta (1-42) (*i.e.* Aβ (1-42)) is the preferred inducing stimulus, any other amyloid beta fragments such as (1-39), (1-40), (1-41), (1-43), (25-35), (16-22), (16-35), (10-35), (8-25), (28-38), (15-39), (15-40), (15-41), (15-42), (15-43) or any other amyloid beta fragment may also be used in any of the methods or use of kits described herein.

The following examples are provided by way of illustration to further describe certain preferred embodiments of the invention

## EXAMPLES

The Reference Examples are useful for understanding the invention but are outside its scope

## REFERENCE EXAMPLE 1: Alzheimer's Disease-Specific Molecular Biomarker (ADSMB)

Bradykinin (10 nM, 10 min at 37°C) was found to cause greater phosphorylation of Erk1/2 in Alzheimer's (AD) fibroblasts vs. non-AD dementia and non-demented control fibroblasts. While this increased Erk1/2 phosphorylation for AD fibroblasts could be observed here with additional Coriell Cell lines, the inherent variability found in these measurements indicated a need for improved quantitation, reliability, and reproducibility. Here, therefore, to control for intrinsic differences in growth rates of the fibroblast cell lines, as well as differences in the exact quantities of protein extracts applied to the gels, we introduce a new measure of phosphorylation-one that compares Erk1 to Erk2 phosphorylation in every patient sample using a Erk1/2 ratio before and after BK+ stimulation. This measure of Erk1/Erk2 phosphorylation ratio, the Alzheimer's Disease-Specific Molecular Biomarker (ADSMB), completely distinguished all non-demented control fibroblasts from all AD fibroblasts (Figures 1 and 4A). The apparent higher level of p-Erk1 for control cases (BK-) (Figure 3) was not consistent for all patients. A few cases of non-AD dementia were not distinguished, although these can be due to the lack of autopsy confirmation of the clinical diagnoses. This interpretation was supported by the results of the ADSMB measurement obtained with fibroblasts from patients with autopsy-confirmed diagnoses (Figures 1 and 4B), in which the ADSMB accurately distinguished all AD cases from all non-AD dementias and even cases of "mixed" dementia due to both AD and other non-AD etiologies such as Parkinson's disease. This high accuracy in distinguishing AD from both non-AD dementia and non-demented control patients is reflected in the remarkable sensitivity and specificity of the ADSMB (Figure 6) considering both the Coriell cell samples and the autopsy-confirmed samples. When only the autopsy-confirmed diagnoses are considered, sensitivity and specificity are at the 100% levels.

Alzheimer's Disease-Specific Molecular Biomarker (ADSMB) Varies with Disease Duration

For a sample of those patients for whom disease duration was available (i.e. time of ADSMB measurement from the time of symptom(s) onset), we examined the relation of ADSMB amplitudes to disease duration. As illustrated (Figure 2), there was a significant (with linear regression analysis) inverse correlation of ADSMB magnitude with disease duration. These results suggest that the MAP Kinase phosphorylation ADSMB is more marked, the earlier time in the course of the disease at which it is measured.

Induction of Alzheimer's Phenotype by AB (1-42)

Because $A\beta(1-42)$ levels are most likely to be critically involved in early AD, and because the observed ADSMB was shown by the data to have early AD diagnostic power, we examined here the possibility that elevated $A\beta(1-42)$ might induce abnormalities of MAP kinase D.F. Fibroblast cell lines from normal control patients, therefore, were exposed for 24 hours to $1.0\mu M$ $A\beta(1-42)$. As illustrated in Figure 5A, preincubation with $A\beta(1-42)$ did, as predicted, convert the normal (negative) ADSMB phenotype into the abnormal positively valued ADSMB phenotype that had been observed for all of the AD phenotypes. These results suggest that this ADSMB phenotype in AD patients actually arose from elevated levels of $A\beta(1-42)$.

Reversal of Alzheimer's Phenotype by the PKC activator, Bryostatin

As discussed earlier, MAP Kinase phosphorylation (measured by the ADSMB) is regulated by PKC activation that, in turn showed vulnerability to elevated levels of $A\beta$. Furthermore, the potent PKC activator, the macrolactone, Bryostatin, was found to enhance PKC activation in human fibroblasts as well as to reduce $A\beta(1-42)$ levels in the brains of transgenic mice with human AD genes. Based on these findings, therefore, we tested the effects of Bryostatin (0.1 nM) on $A\beta(1-42)$-treated human fibroblasts. As illustrated (Figure 5B and Table 1), Bryostatin entirely reversed the change of MAP Kinase phosphorylation induced in normal fibroblasts by $A\beta(1-42)$. Bryostatin changed the abnormal, positively valued of $A\beta$-treated fibroblasts into the normal, negatively valued ADSMB previously observed for non-AD fibroblasts. This "therapeutic" efficacy of Bryostatin is consistent with the greatly increased survival of AD-transgenic mice that were exposed to chronic Bryostatin treatment.

Table 1

| Alzheimer's Disease-Specific Molecular Biomarker (ADSMB) was measured for AC (control cell lines) (control), amyloid beta ($A\beta$) induced cells and, amyloid beta ($A\beta$) induced cells plus bryostatin treatment ($A\beta$+BY). | | | |
| --- | --- | --- | --- |
| Cell lines | ADSMB[a] | | |
| | Control[*] | $A\beta$[*#] | $A\beta$=BY[#] |
| AG06959 | - | 0.13 | 0.0 |
| AG07732 | -0.11 | 0.12 | -0.13 |
| AG 11363 | 0.0 | 0.16 | 0.09 |

(continued)

| Alzheimer's Disease-Specific Molecular Biomarker (ADSMB) was measured for AC (control cell lines) (control), amyloid beta (Aβ) induced cells and, amyloid beta (Aβ) induced cells plus bryostatin treatment (Aβ+BY). | | | |
|---|---|---|---|
| Cell lines | ADSMBa | | |
| | Control* | Aβ*# | Aβ=BY# |
| AG09977 | -0.15 | 0.13 | 0.01 |
| Average ± SE | -0.09±0.05 | 0.13±0.01 | -0.01±0.05 |
| *P<0.001, #P<0.01 aADSMB was calculated according to method use by this study. *T-test was conducted between control and AP treated cells. #T-test was conducted between AP treated cells and AP plus bryostatin treated cells. | | | |

The high sensitivity and specificity of the ADSMB measure of MAP Kinase phosphorylation to diagnose AD suggest an important potential as a laboratory test for AD to aid in the clinical assessment of dementia. To date, autopsy confirmation of clinically-diagnosed dementia is usually available only for patients with long-standing disease. Considering that AD can last for 8-15 years, clinical diagnosis for AD of brief duration has been found to show high inaccuracy when it is compared to clinical diagnosis later in the disease progression and then subjected to autopsy validation. Thus a peripheral biomarker, here a MAP kinase phosphorylation ratio for human fibroblasts, has real utility in arriving at therapeutic strategies for dementia.

Although not wishing to be bound by theory, it is also of interest to consider why the ratio of Erk1 to Erk2 phosphorylation might be sensitive to abnormalities due to AD-specific differences of Aβ metabolism. One implication of this and past studies of peripheral biomarkers for AD is that the pathophysiology of AD does not only involve the brain, but also a variety of other organ systems. This systemic pathophysiologic view of AD is consistent with observations that amyloid and tau metabolic pathways are ubiquitous in the human body and manifest in blood, saliva, skin and extra-brain tissues.

The close correlation shown here of the Erk1/Erk2 ratio with AD also focuses attention on these substrates as a "read-out" of AD signaling. For example, PKC isozymes regulate several molecular targets that converge on MAP Kinase. PKC activates: (1) α-secretase increase of s-APP and, thus, indirectly, reduction of β-amyloid; (2) β-amyloid activates glycogen synthase Kinase-3β (GSK-3β) that increases MAP kinase Phosphorylation; (3) PKC inhibits GSK-3β; (4) PKC itself phosphorylates GSK-3β; and (5) PKC activates cytokines inflammatory signals that may respond to BK and other AD-initiated events; (6) Toxic cholesterol metabolites (e.g. 17-OH cholesterol) inhibit PKC α that, on balance reduces Aβ, and reduces phosphorylated tau.

Evidence that dysfunction of PKC isozymes themselves may contribute to the earliest initiation of the AD process, therefore, reflected, via all of the above signaling events, in abnormality of the Erk1/2 phosphorylation ratio.

Finally, it is also a mystery as to how the specificity of AD phosphorylation abnormality may be maintained through the successive passages of human fibroblast cell lines. This phenomenon might be accounted for through an interaction of PKC/MAP kinase levels with the fibroblast genome. It is known that PKC and MAP kinase regulate gene expression. It may be possible, therefore, that an ongoing cycle of PKC/MAP Kinase stimulation of their own synthesis could perpetuate the abnormalities of PKC levels and MAP Kinase phosphorylation from one generation of human fibroblasts to the next.

**REFERENCE EXAMPLE 2: Aβ treatment**

Preparation of Aβ(1-42) solution: Initially 1 mg of Aβ(1-42) was dissolved in hexa-fluoroisopropanol (Sigma, St. Louis, MO) at a concentration of 3 mM and separated into aliquots in sterile microcentrifuge tubes. Hexa-fluoroisopropanol was removed under vacuum and lyophilized. The Aβ (1-42) films were stored at -20°C under dry conditions until use. 5 mM Aβ(1-42) stock solution was prepared from the stored Aβ(1-42) in DMSO just before the experiment. 1.0 μM stock solution was prepared in DMEM medium (supplemented with 10% serum and penicillin/streptomycin) by dissolving Aβ(1-42) from a DSMO stock solution. DMEM medium containing 1.0 μM Aβ(1-42) was added to non-AD control (AC) cells at 90-100% confluence stage in 25 mL cultured flask and kept at cell culture incubator (at 37°C with 5% $CO_2$) for 24 hrs. Cells were 'starved' in serum free medium (DMEM) for 16 hours. 10nM bradykinin (in DMSO) solution was prepared in DMEM medium with 10% serum. 7 mL of 10 nM BK solution were added to the 25 mL cultured flask and incubated at 37°C for 10 min. For the controls, the same amount of DMSO was added in DMEM medium with 10% serum. 7 mL of this medium with DMSO (<0.01%) were added to the 25 mL cultured flask and incubated at 37°C for 10 min. After washing four times with cold (4°C) 1X PBS, flasks were kept in a dry ice/ethanol mixture for 15 min. Flasks were removed from the dry ice/ethanol mixture and then 100 $\mu$L of lysis buffer (10 mM Tris, pH 7.4, 150 mM NaCl, 1 mM EDTA, 1 mM EGTA, 0.5% NP-40, 1% Triton X-100, 1% protease inhibitor cocktail, 1% ser/thr/tyrosine phosphatase inhibitor cocktails) was added into each flask. Flasks were kept on an end-to end shaker in a cold room (4°C) for 30 min and cells were collected from each flask with a cell scraper. Cells were sonicated and then centrifuged at 14000 rpm for 15 min, and the supernatant was used for Western blotting after total protein assay. Total Erk1, Erk2 and the phosphorylated forms of Erk1 and Erk2 (p-Erk1, p-Erk2) were determined using specific antibodies: anti- regular Erk1/2 and anti-phospho Erk1/2. At least three bradykinin treated flasks (BK+) and correspondingly three control flasks (BK-) were included for each cell line to minimize errors in measurement.

Bryostatin treatment

0.1 nM bryostatin solution was prepared in regular DMEM medium (supplemented with 10% serum and penicillin/streptomycin) from DMSO stock solution. After A$\beta$ treatment, cells were washed four times with regular culture medium (supplemented with 10% serum and penicillin/streptomycin). 0.1 nM bryostatin was added to cells and culture flasks were kept in the cell culture incubator (at 37°C with 5% $CO_2$) for 20 min. After five times washing with serum free medium the flasks were kept in an incubator (at 37°C with 5% $CO_2$) in serum free condition for 16 hrs. The Bradykinin induced MAPK assay was done as discussed above.

Data analysis

Signals of the Western blot protein bands were scanned with a Fuji LAS-1000 Plus scanner. The intensity of Erk1, Erk2, p-Erk1 and p-Erk2 were measured from scanned protein bands by a specially designed software developed by Dr. Nelson in our Institute (Blanchette Rockefeller Neurosciences Institute, Rockville, MD). The intensity was measured by strip densitometry. The protein bands were selected by strip and each pixel density was calculated after background subtraction by the software. The ratios of p-Erk1/p-Erk2 were calculated from sample (BK+) and control (BK-) respectively. The following formula was used to distinguish between AD and non-AD cases:

$$\text{ADSMB} = [\text{p-Erk1/p-Erk2}]^{\text{BK+}} - [\text{p-Erk1/p-Erk2}]^{\text{BK-}}$$

$$\text{ADSMB} = \text{Alzheimer's Disease-Specific Molecular Biomarker}$$

**EXAMPLE 3:** *In Vitro* **Assay of Skin Fibroblasts to Determine Ratio of Phosphorylated Erk1 to Phosphorylated Erk2**

Banked skin fibroblasts cells (Alzheimer's Disease (AD), non AD dementia (non-ADD) (e.g. Huntington and Parkinson disease and Clinical Schizophrenia) and age- matched control cells (AC), from Coriell Institute of Medical Research were cultured to 90-100% confluence stage. Cells were "starved" in serum-free medium (DMEM) for 16 hours. 10 nM of Bradykinin (BK) in DMSO in regular medium was added at 37 °C for 0 and 10 min. For the controls, the same amount of DMSO was added.

[After washing four times with cold (4 °C) 1X PBS, flasks were kept in dry ice/ethanol mixture for 15 min. Flasks were removed from dry ice/ethanol mixture and then 80 $\mu$L of lysis buffer (10 mM Tris, pH 7.4, 150 mM NaCl, 1 mM EDTA, 1 mM EGTA, 0.5% NP-40, 1% Triton X-100, 1% protease inhibitor cocktail, 1% ser/thr/tyrosine phosphatase inhibitor cocktails) was added into each flask.

Flasks were kept on an end-to-end shaker in a cold room (4 °C) for 30 min and cells were collected from each flask with a cell scraper. Cells were sonicated and then centrifuged at 14000 rpm for 15 min, and the supernatant was used for Western blotting after total protein assay.

Total Erk1, Erk2 and the phosphorylated forms of Erk1 and Erk2 (p-Erk1, p-Erk2) were determined using specific antibodies: anti-regular Erk1/2 and anti-phospho Erk1/2.

**EXAMPLE 4: Skin Fibroblasts**

Banked skin fibroblasts from patients with AD and age-matched controls are purchased from the Coriell Institute for Medical Research. Autopsy confirmed skin fibroblasts are obtained separately. Patients may be clinically affected with severe dementia, progressive memory loss, and other impaired cognitive functions. Brains from these patients show abnormal EEG and different degrees of cerebral atrophy by CAT or CT scan. Cells from normal individuals with close age matches are used as controls.

Fresh-taken skin fibroblasts. The collection and culture of fibroblasts from freshly obtained skin tissue is performed as follows: Punch-biopsy skin tissues from non-FAD (nFAD) patients and age-matched controls are obtained by qualified personnel. All patients (or representatives) sign informed consent forms.

Banked fibroblasts from Huntington's disease. These fibroblasts are from Huntington's disease (HD) patients, with dementia accompanying typical Huntington's disease symptoms. Fibroblasts from normal age-and gender-matched individuals are used as controls.

**EXAMPLE 5: Materials**

DMEM is purchased from Gibco BRL. Fetal bovine serum is purchased from Bio Fluids. Bradykinin, diphenylboric acid 2-aminoethyl ester (2ABP), protease, and phosphatase inhibitor cocktails are purchased from Sigma; bisindolylmaleimide-1 and LY294002 are purchased from Alexis; PD98059 is purchased from Cell Signaling Technology. Anti-phospho-Erk1/2 antibodies are purchased from Cell Signaling Technology. Anti-regular Erk1/2 is purchased from Upstate Biotechnology. SDS mini gels (4-20%) are purchased from Invertrogene-Novex. Nitrocellulose membranes are purchased form Schleicher & Schuell (Keene, NH). All the SDS electrophoresis reagents are purchased from Bio-Rad. The SuperSignal chemilumines-cence substrate kit is purchased from Pierce.

Alternatively, Bradykinin (M.Wt. 1060.2) was purchased from Calbiochem (San Diego, CA). Anti phospho-p44/p42 MAPK from rabbit was obtained from Cell Signaling Technology (Danvers, MA). Anti-regular Erk1/2 was purchased from Upstate Biotechnology, (Charlottesville, VA), Anti-rabbit secondary antibody was purchased from Jackson Lab (Bar Harbor, ME). Beta amyloid (1-42) (M.Wt. 4514.1) was procured from American Peptide (Sunnyvale, CA). Bryostatin was purchased from Biomol (Plymouth Meeting, PA).

**EXAMPLE 6: Culture of AC and AD Fibroblast Cells**

Banked fibroblasts from Alzheimer's Disease patients including both FAD and nFAD types, and from age-matched controls (AC), are maintained and cultured in T25/T75 flasks with DMEM containing 10% fetal bovine serum (FBS). Cells are used within passages 6 to 17.

**EXAMPLE 7: Processing and Culture of Fibroblasts from Fresh Biopsy Tissue or Banked Samples**

Samples are placed in 1 xPBS and transported in transfer medium to the laboratory for propagation. After the transfer medium is removed, the skin tissues are rinsed with PBS and finely chopped into 1-mm-sized explants. The explants are transferred one by one onto the growth surface of vented T25 flasks with 3 ml of biopsy medium containing 45% FBS and 100 U/ml penicillin and 100 U/ml streptomycin (Pen/Strep). The tissues are cultured at 37 C for 24 h before addition of 2 ml of biopsy medium containing 10% FBS. The medium is replaced after 48 h with 5 ml of regular culture medium containing 10% FBS and 100 U/ml Pen/Strep. The cells are then passaged and maintained according to a regular procedure given above.

Human skin fibroblast cell culture systems have also been used for these studies. Banked skin fibroblasts cells Alzheimer's Disease (AD), non AD dementia (e.g. Huntington and Parkinson disease and Clinical Schizophrenia and age- matched control, AC) from Coriell Institute of Medical Research (Camden, New Jersey) were cultured (supplemented with 10% serum and penicillin/streptomycin, 37°C with 5% $CO_2$) to 90-100% confluence stage in 25mL cell cultured flask. Cells were 'starved' in serum free medium (DMEM) for 16 hours. 10nM bradykinin (in DMSO) solution was prepared in DMEM medium with 10% serum. 7mL of 10nM BK solution was added to the 25mL cultured flask and incubated at 37°C for 10 min. For the controls, the same amount of DMSO was added in DMEM medium with 10% serum. 7mL of this medium with DMSO (<0.01%) was added to the 25mL cultured flask and incubated at 37°C for 10 min. After washing four times with cold (4°C) 1X PBS, flasks were kept in dry ice/ethanol mixture for 15 min. Flasks were removed from dry ice/ethanol mixture and then 100µL of lysis buffer (10mM Tris, pH 7.4, 150mM NaCl, 1mM EDTA, 1mM EGTA, 0.5% NP-40, 1% Triton X-100, 1% protease inhibitor cocktail, 1% ser/thr/tyrosine phosphatase inhibitor cocktails) was added into each flask. Flasks were kept on an end to end shaker in a cold room (4°C) for 30 min and cells were collected from each flask with a cell scraper. Cells were sonicated and then centrifuged at 14000 rpm for 15 min, and the supernatant was used for Western blotting after total protein assay.

**EXAMPLE 8: Treatment of Fibroblast Cells with Different Protein Kinase C Activators**

Bradykinin or different specific protein kinase C activators are used to treat fibroblasts. Banked AC and AD skin fibroblasts are cultured to 80-100% confluence before they are "starved" in serum-free DMEM overnight. Cells are treated with 10 nM protein kinase C activator at 37 C for different lengths of time to establish a time course for the protein kinase C activator-induced effects. The time point at which reactions are terminated immediately after application of protein kinase C activator is defined as "0 min" post-protein kinase C activator treatment. A control flask of cells for each cell line at each treatment time point is added with the identical volume of PBS. The reaction is terminated by removing the culture medium, rapidly rinsing the cells with precooled PBS, pH 7.4, and transferring the flask onto dry ice/ethanol. For cells obtained and cultured from fresh biopsy tissues, a concentration of 0.1 nM protein kinase C activator may be used. The treatment time is about 10 min at 37 C.

To prepare cell lysates from the treated cells, flasks are moved from dry ice/ethanol onto water ice. To each flask is added 1 ml of lysis buffer containing 10 mM Tris, pH 7.4, 150 mM NaCl, 1 mM EDTA, 1 mM EGTA, pH 8, 0.5% NP-40, 1% Triton X-100, 1% protease inhibitor cocktail (Sigma), 1% Ser/Thr, and tyrosine phosphatase inhibitor cocktails (Sigma). After rocking on an end-to-end shaker in a cold room for 30 min, cells are collected from each flask with a cell scraper. Cells are sonicated and centrifuged at 5000 rpm for 5 min, and the supernatant used for Western blotting.

## EXAMPLE 9: Western Blotting

Protocol 1: Cell lysates are treated with an equal volume of 2X SDS-sample buffer and boiled for 10 min. Proteins from each sample are resolved on a 4-20% mini-gradient gel and transferred onto a nitrocellulose membrane. Phosphorylated Erk1/2 is detected with an anti-phospho-Erk1/2 antibody using the SuperSignal ECL detection kit. In order to normalize the amount of phosphorylated Erk1/2 against the total amount of Erk1/2, after being blotted with an anti-phospho-Erk1/2 antibody, the same membrane is stripped with a stripping buffer containing 62.5 mM Tris-HCI, pH 6.7, 2% SDS, and 100 mM 2-mercaptoethanol at 60 C for 45 min and then blotted with an anti-regular Erk1/2 antibody. Alternatively, duplicate samples resolved on SDS-PAGE and transferred to a nitrocellulose membrane are respectively blotted with anti-phospho- and anti-regular Erk antibodies. After being washed with 10 mM PBS, pH 7.4, containing 0.01% Tween 20 (three times for 10 min), the membrane is blotted with an anti-regular Erk1/2 antibody, from which the total amount of Erk1/2 loaded on the SDS gel is measured.

Protocol 2: Equal volumes of 2xSDS sample buffer were added to each cell lysate, and boiled for 10 minutes in boiling water bath. Electrophoresis was conducted on an 8-16% mini-gradient gel and transferred onto a nitrocellulose membrane. Total Erk1, Erk2 and the phosphorylated forms of Erk1 and Erk2 (p-Erk1, p-Erk2) were determined using specific antibodies.

## EXAMPLE 10: Data Analysis

Signals for both phosphorylated and regular forms of Erk1/2 are scanned with a Fujifilm LAS-1000 Plus scanner. The mean optical density of each protein band is measured using NIH Image software. Values from the phospho-Erk1/2 signals are normalized respectively against those of the total Erk1/2 signals. After normalization, data from each treated cell line is converted to a percentage of the basal control and subjected to statistical analyses.

## EXAMPLE 11: Immunocytochemistry

Fibroblast cells are grown on the surface of 2.5-cm-diameter glass coverslips coated with 0.02 mg polylysine. Upon treatment with bradykinin or another protein kinase C activator as described above, cells are rapidly rinsed with cold PBS, pH 7.4, and fixed with 4% formaldehyde in PBS, pH 7.4, at room temperature for 15 min. After being washed with PBS, pH 7.4, three times, each lasting 5 min, cells are penetrated with 0.1% Triton x-100 in PBS, pH 7.4, at room temperature for 30 min. After incubation with 10% normal horse serum in PBS, pH 7.4, at room temperature for 30 min, cells are incubated with anti-phospho-Erk1/2 antibody (1:200) at 4°C overnight. Cells on the coverslips are washed with PBS, pH 7.4, three times and then an anti-mouse IgG labeled with fluorescein (Vector Laboratories) is added (1:200) and incubated with the cells at room temperature for 60 min. Following three washes with PBS, and sealing with Vectashield (Vector Laboratories), immunostaining signals in the cells are observed with a Nikon fluorescene microscope. The intensity of the immunocytochemistry signals in the cell images is measured with Bio-Rad Quantity One software (BioRad) and Tnimage. For localization of the BK or protein kinase C activator receptors in the skin fibroblasts, a monoclonal anti-BK B2 antibody, or anti protein kinase C activator antibody is applied to the normal fibroblasts, followed by incubation with Cy5-conjugated anti-mouse IgG. The resulting immunoreactive signals are imaged with a fluorescence microscope.

## Claims

1. Use of a kit for diagnosing the presence or absence of Alzheimer's Disease in a subject, the kit comprising:

a) an agent that is a protein kinase C activator chosen from bradykinin; $\alpha$-APP modulator; bryostatin; bryologues; bombesin; cholecystokinin; thrombin; prostaglandin F2$\alpha$; vasopressin; 6-[N-decylamino]-4-hydroxymethylinole (DHI); 1,2-Dioctanoyl-sn-glycerol; farnesylthiotriazole (FTT); Gnidimacrin, *Stellera chamaejasme* L.; (-)-Indol-actam V; Lipoxin A$_4$; Lyngbyatoxin A, *Micromonospora* sp.; Oleic acid; 1-Oleoyl-2-acetyl-sn-glycerol; 4$\alpha$-Phorbol-12,13-didecanoate; L-$\alpha$-Phosphatidylinositol-3,4-bisphosphate, Dipalmitoyl-, Pentaammonium Salt; L-$\alpha$-Phosphatidylinositol-4,5-bisphosphate, Dipalmitoyl-, Pentaammonium Salt; L-$\alpha$-Phosphatidylinositol-3,4,5-trisphosphate, Dipalmitoyl-, Heptaammonium Salt; 1-Stearoyl-2-arachidonoyl-sn-glycerol; Thymeleatoxin, *Thymelea hirsuta* L.; insulin; lysophosphatidylcholine; lipopolysaccharide; daunorubicin; and vanadyl sulfate;
b) an antibody specific for a phosphorylated first MAP kinase protein, wherein the first MAP kinase protein is Erk1;
c) an antibody specific for a phosphorylated second MAP kinase protein, wherein the second MAP kinase protein is Erk2;

wherein the use comprises:

i) contacting cells from said subject with said agent that is a protein kinase C activator;
ii) measuring the ratio of said phosphorylated first MAP kinase protein to said phosphorylated second MAP kinase protein, wherein said phosphorylated first and second MAP kinase proteins are obtained from said cells after said contacting step;
iii) measuring the ratio of said phosphorylated first MAP kinase protein to said phosphorylated second MAP kinase protein in cells from said subject that have not been contacted with the agent of step (i);
iv) subtracting the ratio obtained in step (iii) from the ratio obtained in step (ii); and
v) diagnosing the presence of Alzheimer's Disease in said subject based on the difference calculated in step (iv).

2. The use according to claim 1, wherein said protein kinase C activator is selected from the group consisting of bradykinin, bryostatin, bombesin, cholecystokinin, thrombin, prostaglandin F2$\alpha$ and vasopressin.

3. The use according to claim 1, the kit further comprising an amyloid beta peptide.

4. The use according to claim 3, wherein said amyloid beta peptide is A$\beta$ (1-42).

**Patentansprüche**

1. Verwendung eines Kits zum Diagnostizieren des Vorliegens oder der Abwesenheit von der Alzheimer-Krankheit in einem Subjekt, wobei das Kit das Folgende umfasst:

a) ein Mittel, das ein Proteinkinase-C-Aktivator ist, der aus Bradykinin; $\alpha$-APP-Modulator; Bryostatin; Bryologa; Bombesin; Cholecystokinin; Thrombin; Prostaglandin F2$\alpha$; Vasopressin; 6-[N-decylamino]-4-hydroxymethylinol (DHI); 1,2-Dioctanoyl-sn-glycerol; Farnesylthiotriazol (FTT); Gnidimacrin, *Stellera chamaejasme* L.; (-)-Indolactam V; Lipoxin A$_4$; Lyngbyatoxin A, *Micromonospora* sp.; Ölsäure; 1-Oleoyl-2-acetyl-sn-glycerol; 4$\alpha$-Phorbol-12,13-didecanoat; L-$\alpha$-Phosphatidyl-inositol-3,4-bisphosphat, Dipalmitoyl-, Pentaammonium-Salz; L-$\alpha$-Phosphatidylinositol-4,5-bisphosphat, Dipalmitoyl-, Pentaammonium-Salz; L-$\alpha$-Phosphatidylinositol-3,4,5-triphosphat, Dipalmitoyl-, Heptaammonium-Salz; 1-Stearoyl-2-arachidonoyl-sn-glycerol; I; Thymeleatoxin, *Thymelea hirsuta* L.; Insulin; Lysophosphatidylcholin; Lipopolysaccharid; Daunorubicin; und Vanadylsulfat ausgewählt ist;
b) einen Antikörper, der für ein phosphoryliertes erstes MAP-Kinaseprotein spezifisch ist, wobei das erste MAP-Kinaseprotein Erk1 ist;
c) einen Antikörper, der für ein phosphoryliertes zweites MAP-Kinaseprotein spezifisch ist, wobei das zweite MAP-Kinaseprotein Erk2 ist;

wobei die Verwendung das Folgende umfasst:

(i) In-Kontakt-Bringen von Zellen aus dem Subjekt mit dem Mittel, das ein Proteinkinase-C-Aktivator ist;
(ii) Messen des Verhältnisses von dem phosphorylierten ersten MAP-Kinaseprotein zu dem phosphorylierten zweiten MAP-Kinaseprotein, wobei das phosphorylierte erste und zweite MAP-Kinaseprotein aus den Zellen nach dem Schritt des In-Kontakt-Bringens erhalten wurden;
(iii) Messen des Verhältnisses von dem phosphorylierten ersten MAP-Kinaseprotein zu dem phosphorylierten zweiten MAP-Kinaseprotein in Zellen aus dem Subjekt, die nicht mit dem Mittel aus dem Schritt (i) in Kontakt gebracht worden sind;

(iv) Subtrahieren des Verhältnisses, das in dem Schritt (iii) erhalten wurde, von dem Verhältnis, das in dem Schritt (ii) erhalten wurde; und

(v) Diagnostizieren des Vorliegens von der Alzheimer-Krankheit in dem Subjekt, basierend auf der Differenz, der in dem Schritt (iv) berechnet wurde.

2. Verwendung nach Anspruch 1, wobei der Proteinkinase-C-Aktivator aus der Gruppe ausgewählt ist, bestehend aus Bradykinin, Bryostatin, Bombesin, Cholecystokinin, Thrombin, Prostaglandin F2$\alpha$ und Vasopressin.

3. Verwendung nach Anspruch 1, wobei das Kit ferner ein Amyloid-beta-Peptid umfasst.

4. Verwendung nach Anspruch 3, wobei das Amyloid-beta-Peptid A$\beta$ (1-42) ist.

## Revendications

1. Utilisation d'un kit pour le diagnostic de la présence ou de l'absence de la maladie d'Alzheimer chez un sujet, le kit comprenant :

   a) un agent qui est un activateur de la protéine kinase C choisi parmi la bradykinine ; le modulateur d'$\alpha$-APP ; la bryostatine ; les bryologues ; la bombésine ; la cholécystokinine ; la thrombine ; la prostaglandine F2$\alpha$ ; la vasopressine ; le 6-[N-décylamino]-4-hydroxyméthylinole (DHI) ; le 1,2-dioctanoyl-sn-glycérol ; le farnésylthiotriazole (FTT) ; la gnidimacrine, *Stellera chamaejasme* L. ; le (-)-indolactame V ; la lipoxine A$_4$ ; la lyngbyatoxine A, espèce *Micromonospora ;* l'acide oléique ; le 1-oléoyl-2-acétyl-sn-glycérol ; le 4$\alpha$-phorbol-12,13-didécanoate ; le L-$\alpha$-phosphatidylinositol-3,4-bisphosphate, sel de dipalmitoyl-pentaammonium ; le L-$\alpha$-phosphatidylinositol-4,5-bisphosphate, sel de dipalmitoyl-pentaammonium ; le L-$\alpha$-phosphatidylinositol-3,4,5-trisphosphate, sel de dipalmitoyl-heptaammonium ; le 1-stéaroyl-2-arachidonoyl-sn-glycérol ; la thyméléatoxine, *Thymelea hirsuta* L. ; l'insuline ; la lysophosphatidylcholine ; un lipopolysaccharide ; la daunorubicine ; et le sulfate de vanadyle ;

   b) un anticorps spécifique d'une première protéine MAP kinase phosphorylée, dans lequel la première protéine MAP kinase est Erk1 ;

   c) un anticorps spécifique d'une deuxième protéine MAP kinase phosphorylée, dans lequel la deuxième protéine MAP kinase est Erk2 ;

   l'utilisation comprenant :

   i) le contact des cellules dudit sujet avec ledit agent qui est un activateur de la protéine kinase C ;

   ii) la mesure du rapport de ladite première protéine MAP kinase phosphorylée à ladite deuxième protéine MAP kinase phosphorylée, lesdites première et deuxième protéines MAP kinases phosphorylées étant obtenues à partir desdites cellules après ladite étape de contact ;

   iii) la mesure du rapport de ladite première protéine MAP kinase phosphorylée à ladite deuxième protéine MAP kinase phosphorylée dans les cellules dudit sujet qui n'ont pas été mises en contact avec l'agent de l'étape (i) ;

   iv) la soustraction du rapport obtenu à l'étape (iii) du rapport obtenu à l'étape (ii) ; et

   v) le diagnostic de la présence de la maladie d'Alzheimer chez ledit sujet sur la base de la différence calculée à l'étape (iv).

2. Utilisation selon la revendication 1, dans laquelle ledit activateur de la protéine kinase C est choisi dans le groupe constitué de la bradykinine, de la bryostatine, de la bombésine, de la cholécystokinine, de la thrombine, de la prostaglandine F2$\alpha$ et de la vasopressine.

3. Utilisation selon la revendication 1, le kit comprenant en outre un peptide bêta amyloïde.

4. Utilisation selon la revendication 3, dans laquelle ledit peptide bêta amyloïde est un A$\beta$ (1-42).

FIG. 1

FIG. 2

FIG. 3

19

FIG. 4B

FIG. 4A

FIG. 5B

FIG. 5A

PERCENTAGE CORRECT

100

75

50

25

0

☐ SENSITIVITY

▣ SPECIFICITY

BIOMARKER
EFFECTIVENESS

FIG. 6A

PERCENTAGE CORRECT

100

75

50

25

0

☐ SENSITIVITY

▣ SPECIFICITY

BIOMARKER
EFFECTIVENESS

FIG. 6B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02067764 A
- WO 03102016 A2
- WO 02067764 A2
- US 6107050 A

### Non-patent literature cited in the description

- **ZHAO et al.** *Neurobiology of Disease,* 2002, vol. 11, 166-183
- **W.Q. ZHAO et al.** *Abstracts of the Annual Meeting of the Society for Neuroscience,* vol. 27 (1), 924
- **W.Q. ZHAO et al.** *Neurobiology of Disease,* vol. 11 (1), 166-183
- **WENDER et al.** *Organic letters,* 12 May 2005, vol. 7 (10), 1995-8
- **WENDER et al.** *Organic letters,* 17 March 2005, vol. 7 (6), 1177-80
- **WENDER et al.** *Journal of Medicinal Chemistry,* 16 December 2004, vol. 47 (26), 6638-44
- Principles and Practice of Immunoassay. Stockton Press, 1991
- Current Protocols in Molecular Biology. John Wiley and Sons, 1987
- Methods in Immunodiagnosis. John Wiley and Sons, 1980
- **CAMPBELL et al.** Methods of Immunology. W.A. Benjamin, Inc, 1964
- Principles and Practice of Immunoassay. Stockton Pres, 1991
- **OELLIRICH, M.** *J. Clin. Chem. Clin. Biochem.,* 1984, vol. 22, 895-904
- **KANG.** *Nature,* 1987, vol. 325, 733-736
- **PONTE et al.** *Nature,* 1988, vol. 331, 525-527
- **TANZI et al.** *Nature,* 1988, vol. 331, 528-530
- **KITAGUCHI.** *Nature,* 1988, vol. 331, 530-532
- **WALSH et al.** *Nature,* 2002, vol. 416, 535-539
- **JOHNSON-WOOD et al.** *Proc. Natl. Acad. Sci. USA,* 1997, vol. 94, 1550
- **FIELDS et al.** Synthetic Peptides: A User's Guide. Freeman & Co, 1992, 77